# EUROPEAN PATENT SPECIFICATION

(11) **EP 3 723 737 B1**
(45) Date of publication and mention of the grant of the patent: **18.05.2022**
(21) Application number: 19725643.1
(22) Date of filing: 11.03.2019
(51) Int. Cl.: A61K 9/20, A61K 31/166, A61K 31/4045, A61K 9/14, C01B 33/145, C01B 33/148

(54) **POROUS SILICA PARTICLES FOR USE IN COMPRESSED PHARMACEUTICAL DOSAGE FORM**
PORÖSE SILICIUMDIOXIDTEILCHEN ZUR VERWENDUNG IN KOMPRIMIERTER PHARMAZEUTISCHER DOSIERUNGSFORM
PARTICULES POREUSES DE SILICE POUR LEUR UTILISATION DANS UNE FORME POSOLOGIQUE PHARMACEUTIQUE COMPRIMÉE

(30) Priority: 11.03.2018 SE 1850267
(43) Date of publication of application: 21.10.2020
(73) Proprietor: Nanologica AB, 151 36 Södertälje (SE)
(72) Inventor: ZHOU, Chunfang, 113 66 Stockholm (SE); XIA, Xin, 124 76 Bandhagen (SE); PASZKIEWICZ, Paulina, 80634 Munich (DE); FEILER, Adam, 165 71 Hässelby (SE); LENNERNÄS, Hans, 756 52 Uppsala (SE); ARRIAGA GARCIA, Nerea, 162 72 Vällingby (SE)
(74) Representative: Swea IP Law AB
(86) International application number: PCT/EP2019/056015
(87) International publication number: WO 2019/175100

(56) References cited:
- EP-A1- 2 251 038
- WO-A1-2012/035074
- WO-A1-2014/078435
- US-A1- 2011 244 031
- US-A1- 2015 174 101

## Description

### Field of the invention

The present invention relates to a compressed pharmaceutical dosage form as set out in the appended claims, comprising one or more active pharmaceutical ingredients, porous particles and optionally one or more pharmaceutically acceptable excipients, whereby the one or more active pharmaceutical ingredients is in admixture with and/or loaded into said particles, and a porosity of the dosage form is between 5% and 29% as measured by Hg intrusion and/or N₂ sorption, and a hardness of the dosage form is more than 5 and less than 30 N.

### Background of the invention

Fast dissolving tablets (FDT) in the oral cavity have become a rapidly growing area in the pharmaceutical industry. Oral delivery is a preferred route for administration of active pharmaceutical ingredients. For active pharmaceutical ingredients (APIs) undergoing first pass metabolism in the gut and/or liver, less variability in the plasma exposure is expected as the first-pass gut-liver extraction is avoided once absorbed from the oral cavity. FDT tablets may also improve patient compliance and convenience, especially for elderly people and children. Fast dissolving tablets are defined by the FDA, USA, as solid dosage forms containing APIs, which tablets disintegrates rapidly, usually within a matter of seconds, when placed upon the tongue. The tablets may disintegrate into smaller particles and/or melt in the mouth from a hard solid structure to a gel like structure that is attached to the oral mucosa and is absorbed across.

In case of sublingual administration, the dosage form is positioned in the mouth cavity, usually under the tongue. The mucosal lining in the mouth is permeable and active pharmaceutical ingredients can pass across the mucosal barrier to enter venous blood vessels circulating under the mucosa lining. Saliva from salivary gland has a pH of 5.5 to 7.0. The mucus is negatively charged and forms a gelatinous film that permits mucoadhesion of disintegrated particles/substances to the mucosal lining. The relative permeability of the mucosa lining together with accessible blood supply permits rapid absorption of an active pharmaceutical ingredient into the blood by sublingual administration and thus a rapid onset of pharmacological action(s). Especially in cases where subsequent gastrointestinal (GI) absorption is not viable, sublingual administration is a preferred alternative. Thus, the sublingual route has several distinct advantages over the enteral and parenteral routes of active pharmaceutical ingredient delivery due to its rich blood supply, rapid onset of action, enhanced bioavailability, avoidance of the first pass and food effects, increased patient compliance, and ease of self-medication.

Once the sublingual tablet is disintegrated, the active pharmaceutical ingredient can dissolve adjacent to the oral mucosa and permeate into the blood. Preferably, the disintegration occurs such that particles and the dissolved active pharmaceutical ingredient are spread over a surface area under the tongue, i.e. the concentration of the active pharmaceutical ingredient should not be too high at one spot in the mouth cavity, e.g. under the tongue. The dissolution rate is improved if the active pharmaceutical ingredient is spread over a surface area under the tongue after disintegration of the dosage form. The dissolution rate and rate and extent of absorption is directly correlated to the surface area of spreading of the particles and dissolved active pharmaceutical ingredient.

Nasal administration has been investigated for many drugs. Nasal administration has the disadvantage of causing irritation in the nose cavity and suffers from patient to patient variability. If patients have a cold or the nasal mucosa is altered by illness or allergies, the amount of drug per dosage form becomes variable. The bioavailability of drug administered via nasal route is often unpredictable, highly variable and often lower than by oral administration.

Active pharmaceutical ingredients can be absorbed by passive diffusion across various epithelia. The rate of passive diffusion of the active pharmaceutical ingredient into the blood depends on molecular mass, solubility in water, hydrophobicity, polar surface area, number of hydrogen bonds, crystal size and form, concentration, temperature, polarity of the active pharmaceutical ingredient, etc.. Lipids form the main barrier in the mucosa lining, which is why non-polar, unionized active pharmaceutical ingredients can cross the mucosa through the lipid layer. Polar or ionized active pharmaceutical ingredients are more easily transported through ion-channels of the intercellular spaces compared to the transcellular route. Active pharmaceutical ingredients that are soluble in the saliva may not be soluble in the lipid layer and vice versa. Other alternatives for absorption are various carrier mediated transport mechanisms and/or by endocytosis.

Several dosage forms for sublingual administration of active pharmaceutical ingredients have been developed and are being used on the market today. These dosage forms or tablets need to fulfill several requirements, such as rapid disintegration of the tablet, good wettability (often through sufficient porosity or use of excipients), palatability (low particle size, taste masking without feeling gritty), not sensitive to moisture, processable (enough mechanical strength and not friable), mucoadhesive, minimum thickness, stability, etc.. Gels have been developed for sublingual use, whereby swells and disintegrates upon contact with saliva such that the active pharmaceutical ingredient inside the gel can penetrate from the gel into the mucous under the tongue. A limitation with these gels is that disintegration is slow and once the API is released from the gel the API is present at one spot under the tongue at relative high concentration, which reduces the overall absorption as the absorbing area is smaller. Most of the active pharmaceutical ingredients is swallowed due to the low disintegration of the gel and the slow absorption. This causes a variation in dosing upon administration that is not acceptable for a pharmaceutical formulation.

Generally, active pharmaceutical ingredients are mixed with pharmaceutical acceptable excipients and compressed into a tablet. Several excipients can be used to improve disintegration of the tablet and improve dissolution of the active pharmaceutical ingredient into the blood. Examples, such as cellulose derivatives and saccharides have been used. Saccharides, such as mannitol, facilitate disintegration and dissolution as well as improve wettability. Crystalline sugars provide mechanical strength and hardness, while amorphous sugars, e.g. mannitol, are sensitive to moisture. Taste masking excipients can be used as well. Silica is another excipient that can be used to improve porosity and thus wettability of a pharmaceutical dosage form. Silica is also used as a glidant and/or lubricant. However, silica is mostly used in small amounts as it is believed to have a negative impact on the mechanical properties of a tablet, i.e. the tablet becomes too hard, which results in increased disintegration time. In WO 2007/076874 it is suggested to include a superdisintegrant in loadable tablets containing porous silica to obtain a suitable disintegration time.

Porosity in the formulation is needed to improve wettability. Water enters the pores in the formulation, which improves disintegration of the dosage form. However, porosity in a dosage form reduces mechanical strength. Porosity may also disappear completely or partly upon compression of the final tablet.

Several preparation methods have been developed to overcome the limitations and problems associated with sublingual dosage forms. Compression molding provides for high porosity but fragile tablets. Manufacturing, handling, packing and transporting of tablets becomes complex, time consuming and expensive. Freeze drying provides for high porosity but poor mechanical strength and results in the same problems mentioned for compression molding. WO2007/076874 discloses a tablet using silicon or silica, whereby the tablet is loaded with an active pharmaceutical ingredient after preparation of the tablet.

Direct compression provides for tablets having low porosity but improved mechanical strength compared to the molding and freeze drying. The manufacturing process is less complex. However, low porosity increases disintegration rate.

Particle size of the active pharmaceutical ingredient and the granules in the tablet are important for reproducibility of the dosage form as well as for palatability. Manufacturing, handling, reproducibility of the dosage form and the size of granules in the tablet may depend on the active pharmaceutical ingredient and excipients used in the tablet as well as the ratio of these components in relation to each other.

For poorly soluble active pharmaceutical ingredient, loading of the ingredient into a porous particle may improve the dissolution and subsequent absorption of the ingredient. Sublingual tablets comprising such loaded particles have been developed, e.g. W. R Grace Ltd, USA, for a poorly soluble active pharmaceutical ingredient. A disadvantage of these tablets is that absorption of the active pharmaceutical ingredient from the particles into the mucous is still relatively slow, which causes a reduced onset of pharmacological action of the drug. The active pharmaceutical ingredient is loaded in the particles as a base-non-ionic compound. For absorption through the mucous under the tongue, the ionic strength of the compound is important. The dissolution tests are done with powder comprising particles loaded with nifedipine dispersion. No test results are available from compressed tablets comprising these loaded particles. Using only the base compound reduces absorption both qualitative and quantitative.

Niosomes have also been tested as a carrier for active pharmaceutical ingredients in sublingual formulations. These lipid-cholesterol vehicles are soft and not suitable for use in a compressed pharmaceutical dosage form. Besides, not any kind of ingredient can be used together with niosomes.

In the sublingual cavity, compared with the gastrointestinal tract, tablets are subjected to minimal physiological agitation, and a limited volume of saliva is available to facilitate disintegration and dissolution. Without good disintegration and dissolution, there is significant risk that the active pharmaceutical formulation is swallowed rather than being absorbed in the oral cavity, which negates much of the purpose of sublingual administration.

### Summary of the invention

It is an objective of the present invention to at least partly overcome the above-mentioned problems, and to provide an improved compressed pharmaceutical dosage form for clinical use in sublingual administration of one or more active pharmaceutical ingredients.

This objective is achieved by a compressed pharmaceutical dosage form as defined in claim 1. The invention relates to a compressed pharmaceutical dosage form comprising or consisting of
one or more active pharmaceutical ingredients present at a concentration between 2 and 60 % w/w based on the total weight of the dosage form,
porous particles, wherein the porous particles have a diameter between 1 and 100 µm, as measured by SEM and the particles are present at a concentration between 5 and 70% w/w based on the total weight of the dosage form and,
optionally one or more pharmaceutically acceptable excipients,
whereby the one or more active pharmaceutical ingredients is in admixture with and/or loaded into said particles, whereby a ratio of unloaded versus loaded ingredient is between 100:1 and 1:100, and
whereby a porosity of the dosage form is between 5% and 29% as measured by Hg intrusion and/or N₂ sorption, and
a hardness of the dosage form is more than 5 N and less than 30 N using a Schleuniger type hardness tester.

The invention further relates to a compressed pharmaceutical dosage form comprising or consisting of one or more active pharmaceutical ingredients present at a concentration between 2 and 60 % w/w based on the total weight of the dosage form, whereby the active pharmaceutical ingredients has a water solubility at 25 °C of 10 mg/ml or more according to Ph.Eur 9th Ed.

In one aspect, the friability value is preferably around 2% or less, or 1% or less. A low friability provides for a dosage form having the required strength.

In a further aspect, the one or more active pharmaceutical ingredients(APIs) is in admixture with said particles. This may be relevant for active pharmaceutical ingredients having a water solubility at 25 °C of 10 mg/ml or more according to Ph.Eur 9th Ed..

In a further aspect, the one or more active pharmaceutical ingredients is loaded into said particles. This may be relevant for active pharmaceutical ingredients having a water solubility at 25 °C of 20 mg/ml or less according to Ph.Eur 9th Ed..

It has been found that an API with good solubility (having a water solubility at 25 °C of 10 mg/ml or more) show an increased dissolution rate from a compressed dosage form when loaded in porous particles compared to when the API is in admixture with the porous particles. This seems especially true when the salt form of an API is loaded in the particles.

In another aspect, the one or more active pharmaceutical ingredients present at a concentration between 2 and 15 % w/w and the particles are present at a concentration between 10 and 50% w/w. In a further aspect, the porosity of the dosage form is more than 5% and less than 30%.

In another aspect, the particles are calcinated. Calcination removed hydroxy groups on the surface of the particles, i.e. both outer surface and pore surfaces., which makes these surfaces more hydrophobic. The calcinated silica often has better handling properties due to less static charging effects compared to silica with high proportion of surface silanol groups. Calcining makes the particles less static and thus easier to handle during manufacturing of the dosage form. A pre-compression step during manufacturing is not needed when the particles are calcinated. This thus reduces manufacturing time and costs.

In another aspect, the porous particles are silica mesoporous particles. In a further aspect, the particles are substantially spherical. A regular shape of the particles makes them easier to handle and improves homogeneity and content of uniformity of the dosage form. Irregular shaped particles are more difficult to handle during the manufacturing process. A regular, e.g. a spherical shape thus improves the efficiency and robustness of the manufacturing process and reduces cost-of-goods and may require less identification of critical process parameters (CPPs) related to manufacturing.

In one aspect, the concentration of porous particles is between 5 and 50% w/w, or between 5 and 40% w/w, or between 10 and 40% w/w, or between 5 and 30% w/w, or between 10 and 30% w/w, , or between 15 and 20% w/w, or about 5% w/w, or about 15% w/w, based on the total weight of the dosage form. In another aspect, the concentration of porous particles is between 10 and 24% w/w. This defined concentration of particles in the dosage form provides for a dosage form that has the required strength as well as wettability. Combined with the other features in the claims, this concentration improves the release of the active pharmaceutical ingredient during and after disintegration in a quick and controlled manner. This concentration also prevents any problems with palatability after disintegration of the dosage form. The total amount of active pharmaceutical ingredient per dosage form may also be reduced, because of the fast disintegration and thus absorption of the active pharmaceutical ingredient. This saves costs and reduces side effects of an active pharmaceutical ingredient.

In an additional aspect, the active pharmaceutical ingredient is loaded into the particles. The porous particles may be loaded with between 2 and 50%, or between 15 and 40% or between 15 and 35%, or between 15 and 25%, of one or more active pharmaceutical ingredient, whereby 100% is the total pore volume porous particles. In yet a further aspect, the active pharmaceutical ingredient is loaded on the particles, i.e. substantially present on the surface of the particles. Any mixtures of these aspects may be applied as well. Loading hereinafter is defined as active pharmaceutical ingredient being loading into the particles.

Loading may not be necessary for active pharmaceutical ingredient that have a water solubility at 25°C of 10 mg/ml or more according to Ph.Eur 9th Ed. and which have a fast dissolution rate, although the results show that loading improves dissolution even for highly soluble ingredient. For water soluble active pharmaceutical ingredient that have a slow dissolution rate, loading can increase the dissolution rate, and silica particles can improve the hardness and disintegration time of the dosage form, which may improve dissolution and subsequent absorption of the active pharmaceutical ingredient in the blood of the user (such as a human patient). Even when the active pharmaceutical ingredient is in admixture with the particles, the dissolution/release kinetics are believed to be improved using porous particles.

In one aspect, the ratio of unloaded versus loaded ingredient is between 1:99 and 50:50. An advantage of such a ratio is that the unloaded active pharmaceutical ingredient becomes available for absorption into the blood immediately during disintegration of the tablet, while the loaded active pharmaceutical ingredient becomes available quickly after this first absorption phase from the dosage form. This allows for a quick first absorption followed by a quick but more steady state absorption of the active pharmaceutical ingredient after disintegration. In other words, the concentration of the active pharmaceutical ingredient in the blood (Cₚₗₐₛₘₐ) will increase during disintegration and spreading and then continue to rise substantially constantly after disintegration. This ratio allows thus for a quicker onset of medical action of the drug as well as a controlled increase in the plasma concentration-time profile for the drug immediate after disintegration. The porous particles combined with the ratio as defined above improve control of release from the dosage form. Such control in release of the active ingredient can also prevent the Cₚₗₐₛₘₐ from rising to toxic levels in the blood where there is an increased risk for adverse effects as well as toxic effects

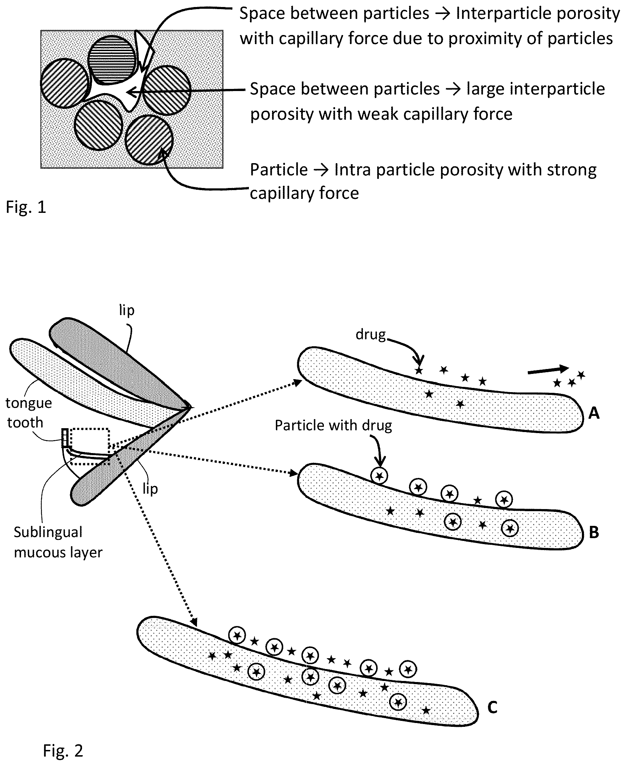

Porous particles have attracted interest in active pharmaceutical ingredient delivery due to a large surface area, high porous volume, controllable pore size and structure and particle size. In this disclosure, a dosage form with high porosity and a combination of different pore structures demonstrated to improve penetration of water into the dosage form due to a large surface and strong capillary forces of the porous particles. As shown in figure 1, the porous particles generate inter and intra porosity. The intraparticle porosity remains even after compression of the dosage form, while the interparticle porosity reduces upon compression of the tablet. The capillary force, driving the imbibition of water is a consequence of the capillary pressure, which is described by the Young-Laplace pressure:
Δp = 2γcosθ/r, where Δp is the capillary pressure, y is the surface tension and cosθ is the contact angle and r is the radius of the pore. It is clearly seen that the capillary pressure increases exponentially with decreasing pore size. Therefore, comparing the capillary pressures resulting in pores formed between particles typically in the micron size range, with the pore sizes of nanometers within the porous particles generates capillary forces orders of magnitude larger due to intraparticle pores compared to interparticle pores. As shown in figure 1, the interparticle spaces have a variation of sizes, which leads to a variation of capillary forces. The largest capillary force occurs at the contact points between the excipient ingredients. The porous particles guarantees a homogenous porosity throughout the dosage form and improves wettability and thus dissolution rate. This is especially important for potent active pharmaceutical ingredients with a narrow therapeutic index and having a long terminal half time (t_{½}), which may cause serious side effects if absorption from a poor dosage form is highly variable and not sufficient robust. The use of the loaded particles allows for a more homogeneous pharmaceutical composition of the dosage form. Due to mixing, the pharmaceutical composition of a dosage form may vary. This variation is reduced by using particles loaded with the API.

The use of porous particles improves the spreading of the dosage form over a larger area under the tongue. This improved dispersion and absorption of the API and provide a more rapid onset of the medical action. Porous particles may decrease tablet content variability by loading active pharmaceutical ingredient in the pore, especially for low-dose and high-potent active pharmaceutical ingredients, and for active pharmaceutical ingredients with needle, rods, or angular morphologies. The porous particles may be nanoporous or mesoporous particles. The use of porous particles in the dosage form of the invention reduces the dependency on polarity on of the active pharmaceutical ingredient. This also reduces manufacturing time and costs.

In one aspect, the active pharmaceutical ingredient is present as a salt thereof. Ionised charge on the ingredient improves absorption of the ingredient into and through the mucous, while the base form penetrated well through the mucous.

The differences between the effect on absorption of the dosage form of the present invention and the dosages forms in the prior art are illustrated in figure 2. Figure 2A illustrates the effect of using a gel. The active pharmaceutical ingredient as released from the gel is located on one spot under the tongue. This reduces the dissolution rate and thus the absorption rate and results in a substantive portion of the ingredient being swallowed. Figure 2B illustrates the effect of using a pharmaceutical composition, whereby the one or more active pharmaceutical ingredients is only present inside the porous particles. As shown in the figure, part of the particles diffuses into the mucosa of the oral cavities. The active pharmaceutical ingredient is being released from the particles in the mouth cavity as well as in the mucous.

Figure 2C illustrates the effect on absorption of the dosage form of the present invention. Due to the presence of particles, the active pharmaceutical ingredient and porosity as defined, the absorption is improved. More ingredient diffuses into the 100 to 200 µm mucus layer and thus more ingredient is being absorbed into the blood. The dosage form of the invention thus has an improved efficacy and efficiency. Embedding of the particles in the mucus layer of the oral cavity reduces the risk of swallowing the active pharmaceutical ingredient. It also allows for a more sustained release of the active pharmaceutical ingredient from the dosage form. Especially for ingredients having a water solubility at 25°C of 10 mg/ml or less according to Ph.Eur 9th Ed., this embedding of the particles improves the absorption of the active pharmaceutical ingredient and thus the efficacy and efficiency of the dosage form. A unique feature of the dosage form of the invention is that the particles are being retained in the mucus, which increases overall absorption.

In one aspect, the porous particles are uncalcinated silica particles. The advantage of uncalcinated silica particles is a more soluble silica since calcination strengthens /sinters the silica and causes removal of hydroxyls. Using uncalcined silica removes the need for further chemical treatment of silica such as chemical etching to render the surface more hydrophilic. In another aspect, the porous particles have a diameter between 1 and 30 µm. In an aspect, the particles do not have a size in the nano range, or below 1 µm.

In one aspect, the porous particles are silica mesoporous particles having a surface area between 200 and 1000 m²/g and/or a pore volume between 0.2 and 3 cm³/g and/or an average pore size between 2 and 50 nm. In a further aspect, the particles are mesoporous silica particles having a diameter between 5 and 20 µm, a surface area between 200 and 850 m²/g, a pore volume between 0.6 and 2 cm³/g and/or an average pore size between 5 and 30 nm. In another aspect, the active pharmaceutical ingredient is in the amorphous state, wherein the porous particles have a surface area of at least 50 m²/g and/or a pore volume of at least 0.2 cm³/g and/or an average pore size between 2 and 250 nm. These particles, when used in the concentration and ratios as defined in the claims, provide for a dosage form in which the active pharmaceutical ingredient is released from the dosage form during disintegration of the dosage form as well as released at a substantially controlled rate from the dosage form after disintegration of the dosage form. These combinations of features improve the ratio of intra-porosity versus inter-porosity and thus provide the required wettability and hardness of the dosage form.

In one aspect, the porosity of the dosage form is above 5% and below 29% as measured by Hg intrusion and/or N₂ sorption. In a further aspect, the porosity of the dosage form is at least 10% or between 5 and 29%, or between 10 and 29%. The higher the porosity the better the wettability of the dosage form, which results in an increased disintegration, dissolution and bioavailability. However, the porosity influences the mechanical strength. A porosity below 29% provides a dosage form that has the required mechanical strength, is relatively easy to manufacture at low cost, while having the required disintegration requirements. In one aspect, a ratio of intra-porosity versus inter-porosity is between 1:2 and 20:1. In another aspect, the ratio of intra-porosity versus inter-porosity is between 0.2:1 and 10:1, or 0.5:1 and 6:1. An optimized and controlled interparticle porosity versus intraparticle porosity in the tablet can provide adequate mechanical integrity without compromising disintegratability. This also improves reproducibility in manufacturing of the dosage form.

The taste of the active pharmaceutical ingredient may be masked, when an active pharmaceutical ingredient is loaded into the pores of the porous particles. This has the advantage that no additional excipients for masking a taste of an active pharmaceutical ingredient are needed. This reduces cost and time for manufacturing.

The dosage form disintegrated within 1 minute, or within 30 seconds after administration under the tongue. After disintegration, the particles and dissolved active pharmaceutical ingredient are spread over a surface area under the tongue, especially in the case when the porous particles are loaded with the active pharmaceutical ingredient. The unloaded active pharmaceutical ingredient can readily be absorbed, while the loaded active pharmaceutical ingredient is first spread out in the mouth cavity after disintegration and then absorbed. This spreading of the porous particles improves the dissolution rate and increases the absorption rate and thus the bioavailability of the active pharmaceutical ingredient compared to when the dosage form disintegrates at one spot under the tongue. This one spot will have a higher local concentration of the active pharmaceutical ingredient, which decreases dissolution rate and thus a lower and more variable absorption and bioavailability of the active pharmaceutical ingredient.

Rapid uptake and absorption by means of sublingual absorption is particularly desirable for rapid or instant and "on demand" effect for example immediate treatment of the symptoms of gastroparesis including nausea and pain, or treatment of pain such as break through pain, or any other form of pain, or treatment of migraines as well as for the ability to overcome erectile dysfunction. Other desirable rapid effects include treatment of insomnia, heart failures, epilepsy or anaphylaxis.

In another aspect, the active pharmaceutical ingredient has a water solubility at 25°C of 10 mg/ml or more according to Ph.Eur 9th Ed.. An example of the active pharmaceutical ingredient may be metoclopramide, which has a solubility of 10 mg/ml in buffer at pH 6.8 and > 6 mg/ml in water. A further example may be melatonin.

In yet another aspect, the active pharmaceutical ingredient has a water solubility at 25°C of 1 to 10 mg/ml according to Ph.Eur 9th Ed..

In an aspect, the active pharmaceutical ingredient has a water solubility at 25 °C below 1 mg/ml according to Ph.Eur 9th Ed.. Porous particles may enhance dissolution kinetics of poorly water-soluble active pharmaceutical ingredients, i.e. active pharmaceutical ingredients having a water solubility at 25°C below 20, or 10, or 1 mg/ml. The dosage form of the present invention is especially suitable for active pharmaceutical ingredient having a water solubility at 25°C of 20 mg/ml or less according to Ph.Eur 9th Ed. that are loaded into the particles. The dosage form of the present invention is especially suitable for active pharmaceutical ingredient having a water solubility at 25°C of 10 mg/ml or 20 mg/ml or more according to Ph.Eur 9th Ed. that are in admixture with the particles.

The active pharmaceutical ingredient may be in amorphous or crystalline form. The active pharmaceutical ingredient can be loaded into the pores of the particles. In another aspect, the active pharmaceutical ingredient is in the amorphous state, wherein porous particles have the surface area at least 500 m²/g and/or a pore volume at least 0.3 cm³/g and/or an average pore size between 2 and 250 nm. The amorphous state may improve absorption of the active pharmaceutical ingredient into the blood as compared to a none-amorphous state of the active pharmaceutical ingredient.

In one aspect, the concentration of the active pharmaceutical ingredient is between 5 and 50 % w/w or between 5 and 40 % w/w, or between 5 and 20 % w/w, between 5 and 15 % w/w based on the total weight of the dosage form.

In another aspect, the active pharmaceutical ingredient is metoclopramide or melatonin. In one aspect, the active pharmaceutical ingredient is metoclopramide. In another aspect, the active pharmaceutical ingredient is melatonin. In a further aspect, the active pharmaceutical ingredient is selected from the group comprising or consisting of metoclopramide, melatonin, loratadine, donepezil, diphenhydramine, pseudoephedrine, lansoprazole, mirtazapine, rizatriptan, ondansetron, zolmitriptan, olanzapine, morphine, erythromycin, domperidone, omeprazole, avanafil, lodenafil, mirodenafil, sildenafil, tadalafil, vardenafil, udenafil, zaprinast, benzamidenafil, nitroglycerine, oestrogen, progesterone, adrenaline and dasantafil. The dosage form of the invention is especially suitable for active pharmaceutical ingredients having a terminal half time (t½) of 6 hours or less, or 2 hours or less.

In yet another aspect, no further pharmaceutically acceptable excipients are present. More than one active pharmaceutical ingredient may be present in the dosage form. In yet another aspect, the compressed pharmaceutical dosage form is for immediate release of the one or more active pharmaceutical ingredient.

In one aspect, the compressed pharmaceutical dosage form comprises or consists of the one or more pharmaceutically acceptable excipient as a binder selected from the group comprising or consisting of cellulose, microcrystalline cellulose, hydroxypropylmethylcellulose, starch and polyvinyl pyrrolidone. In one aspect, the binder is microcrystalline cellulose.

In another aspect, the compressed pharmaceutical dosage form comprises or consists of the one or more pharmaceutically acceptable excipient as a disintegrant selected from the group comprising or consisting of (low-substituted) hydroxypropylmethylcellulose, cross-linked sodium carboxymethylcellulose, crosscarmellose, crosspovidone and sodium starch glycolate. In one aspect, the disintegrant is hydroxypropylmethylcellulose and/or cross-linked sodium carboxymethylcellulose. In one aspect, the disintegrant is hydroxypropylmethylcellulose, which may be low-substituted.

In another aspect, the compressed pharmaceutical dosage form comprises or consists of the one or more pharmaceutically acceptable excipient as a filler selected from the group comprising or consisting of mannitol, lactose, sucrose, magnesium stearate, glucose, plant cellulose and calcium carbonate. In one aspect, the filler is mannitol.

In a further aspect, the compressed pharmaceutical dosage form comprises or consists of the one or more pharmaceutically acceptable excipient being a binder, a disintegrant and a filler, wherein the binder is microcrystalline cellulose, the disintegrant is low-substituted hydroxypropylcellulose and the filler is mannitol. A glidant may be added, which may be magnesium stearate.

The excipient polyvinyl acetate is preferably not use due to irritatability of eyes, nose, throat and lungs and a possible effect on the immune system. Excipients like sodium saccharine are rather avoided because it makes the dosage form unsuitable for diabetic patients. Minor amounts of mannitol can be tolerated by diabetic patients.

In a further aspect, the ratio of binder:disintegrant is between 11:1 and 7:1, or between 10:1 and 8:1. In a further aspect, the ratio of porous particles:binder:disintegrant is between 3:10:1 and 2:8:1. It has been found that this ratio improves the disintegration rate of the dosage form and thus absorption of the active pharmaceutical ingredient into the blood. This ratio also provides a dosage form having the required hardness.

The binder may advantageously have disintegration properties. Likewise, the disintegrant may advantageously have binding properties. The filler may have binding properties.

In one aspect, the binder is microcrystalline cellulose and the disintegrant is hydroxypropylmethylcellulose.

In another aspect, the compressed pharmaceutical dosage form comprising or consisting of one or more active pharmaceutical ingredients present at a concentration between 5 and 15 % w/w based on the total weight of the dosage form,
silica mesoporous particles, wherein the porous particles have a diameter between 1 and 100 µm, as measured by SEM and the particles are present at a concentration between 10 and 40% w/w based on the total weight of the dosage form and,
one or more pharmaceutically acceptable excipients, whereby a ratio of binder:disintegrant is between 10:1 and 8:1, and
whereby the one or more active pharmaceutical ingredients is in admixture with and/or loaded into said particles, whereby a ratio of unloaded versus loaded ingredient is between 50:1 and 1:50, and
whereby a porosity of the dosage form is more than 5% and less than 45% as measured by Hg intrusion and/or N₂ sorption, and
the hardness of the dosage form is more than 15 N and less than 30 N using a Schleuniger type hardness tester and having a friability of 2% or less.

In a further aspect, the porosity of the dosage form is more than 5% and less than 30%.

In one aspect, the binder is microcrystalline cellulose and the disintegrant is hydroxypropylmethylcellulose. In another aspect, the silica particles are calcinated. In a further aspect, the active pharmaceutical ingredient is in admixture with or loaded into said particles. In an aspect, the particles are substantially spherical.

In another aspect, the compressed pharmaceutical dosage form comprising or consisting of one or more active pharmaceutical ingredients, a water solubility at 25°C of 10 mg/ml or less according to Ph.Eur 9th Ed., present at a concentration between 5 and 15 % w/w based on the total weight of the dosage form, silica mesoporous particles, wherein the porous particles have a diameter between 1 and 100 µm, as measured by SEM and the particles are present at a concentration between 10 and 40% w/w based on the total weight of the dosage form and,
one or more pharmaceutically acceptable excipients, whereby a ratio of binder:disintegrant is between 10:1 and 8:1, and
whereby the one or more active pharmaceutical ingredients is in admixture with and/or loaded into said particles or loaded into said particles, whereby a ratio of unloaded versus loaded ingredient is between 50:1 and 15:1, and
whereby a porosity of the dosage form is more than 5% and less than 45% as measured by Hg intrusion and/or N₂ sorption, and
the hardness of the dosage form is more than 15 N and less than 30 N using a Schleuniger hardness type tester, and having a friability of 2% or less.

In a further aspect, the porosity of the dosage form is more than 5% and less than 30%.

In one aspect, the binder is microcrystalline cellulose and the disintegrant is hydroxypropylmethylcellulose. In another aspect, the silica particles are calcinated. In a further aspect, the active pharmaceutical ingredient is in admixture with and loaded into said particles. In an aspect, the particles are substantially spherical.

In another aspect, the compressed pharmaceutical dosage form comprising or consisting of one or more active pharmaceutical ingredients, a water solubility at 25°C of 10 mg/ml or more according to Ph.Eur 9th Ed., present at a concentration between 5 and 15 % w/w based on the total weight of the dosage form,
silica mesoporous particles, wherein the porous particles have a diameter between 1 and 100 µm, as measured by SEM and the particles are present at a concentration between 10 and 40% w/w based on the total weight of the dosage form and,
one or more pharmaceutically acceptable excipients, whereby a ratio of binder:disintegrant is between 10:1 and 8:1, and
whereby the one or more active pharmaceutical ingredients is in admixture with and/or loaded into said particles or loaded into said particles, whereby a ratio of unloaded versus loaded ingredient is between 100:1 and 50:50, and
whereby a porosity of the dosage form is between 5% and 29% as measured by Hg intrusion and/or N₂ sorption, and
the hardness of the dosage form is more than 15 N and less than 30 N using a Schleuniger type hardness tester and having a friability of 2% or less.

In a further aspect, the porosity of the dosage form is more than 5% and less than 30%.

In one aspect, the binder is microcrystalline cellulose and the disintegrant is (low substituted) hydroxypropylmethylcellulose. In another aspect, the silica particles are calcinated. In a further aspect, the active pharmaceutical ingredient is in admixture with and loaded into said particles. In an aspect, the particles are substantially spherical. The dosage form of the present invention with the ingredients, percentages and ratios as defined herein, has enough mechanical strength to be processed, handled and transported at lower costs compared to known dosage forms, while at the same time having enough porosity to improve wettability and thus improve disintegration and dissolution.

In a further aspect, the dosage form is a tablet with a hardness of at least 10 Newton (N) or below 30 N or 1.0 to 3.0 kg/cm² using a Schleuniger type hardness tester. In one aspect, the dosage form is a tablet with a hardness between 15 N and 29 N or between 20 N and 29 N. The dosage form of the present invention has a harness that assures the mechanical strength needed for processing, handling and transporting of the dosage form, without affecting the disintegration rate of the dosage form. Due to the combination of feature in the dosage form as defined above, and the ratios defined above, the dosage form does not need to be harder than 30 N. This prevents a decrease in porosity during manufacturing, which introduces variability between batches and also ensures a porosity below 30%, which results in a wettability that causes a quick disintegration of the dosage form.

In yet a further aspect, the dosage form is a tablet having a disintegration rate of 30 seconds at the most, as measured by USP disintegration apparatus. A fast disintegration improves bioavailability.

In one aspect, the dosage form has a maximum thickness of 5 mm. A dosage form for sublingual administration cannot be too thick, because this would cause a discomfort for the user. It would also increase the distance for the active pharmaceutical ingredient to the mucosa, which would reduce the dissolution rate. The dosage form of the disclosure allows for a minimum thickness by using porous particles that provide high porosity (< 30%) and high pore volume (> 0.2 cm³/g). The dosage form of the disclosure allows for the presence of more active pharmaceutical ingredient per cm³ of dosage form compared to known dosage forms, which in turn results in thinner tablets.

In a further aspect, at least a portion of the total porous particles are coated. Porous particles can be modified with other excipients or coated to modulate (wetting) properties of the dosage form. Porous particles can be modified with muco-adhesive agents to promote retention of the active pharmaceutical ingredient under the tongue, establish a higher local concentration gradient across the epithelial barrier and in the mouth to prevent swallowing the active pharmaceutical ingredient before being absorbed. This would provide a robust absorption with a higher bioavailability. Porous particles can be loaded or coated with taste masking agents. The porous particles have mucoadhesive properties. Thus, especially when the porous particles are loaded, the addition of a mucoadhesive agent may not be necessary. Porous particles may be coated to change the charge on the surface of the particles. This may improve inter particles porosity and thus wettability of the dosage form.

In an aspect, at least a portion of the total porous particles in the dosage form are coated with amino acids. Examples of suitable amino acids may be selected from the group comprising L-lysine, L-alanine, glycine and L-tyrosine.

It may be advantageous to use porous particles of different sizes, and different particles characteristics (pore volume, surface are, pore volume, etc.), manufactured using different methods, coated or not, to improve disintegration and dissolution rate. In one aspect, the porous particles in the dosage form are manufactured using at least two different methods and/or only a portion of the particles is coated. In another aspect, no excipients are present in the dosage form.

Porous particles can also be synthesized using a sol-gel method, such as the Stöber process, a spray drying method and fumed process.

In one aspect, the dosage form is for use in sublingual or buccal administration, whereby the onset of action of the active pharmaceutical ingredient administered sublingually or buccally is faster compared to administration through the gastrointestinal tract. The dosage form may be a tablet. The dosage form may be a film from which the active pharmaceutical ingredient is released and absorbed in the blood. Preferably, such film has adhesion properties allowing the film to adhere or stick to the mucus in the mouth cavity. The dosage form may also be a chewable tablet.

The dosage form may be used for prevention, prophylaxis or treatment of a disease. Examples of diseases, indication or disorders may be allergy, Alzheimer's Disease, cold, sinus infection, ulcers, depression, migraine, nausea, schizophrenia, gastroparesis, insomnia, heart failure, epilepsy, pain, anaphylactic shock and erectile dysfunction. In one aspect, the compressed pharmaceutical dosage form comprises or consists of the active pharmaceutical ingredient metoclopramide for use in prevention, prophylaxis or treatment of a disease, such as gastroparesis. The advantage of sublingual administration of metoclopramide is rapid and direct uptake into the systemic system, avoidance of first pass metabolism and circumventing the need for uptake through gastrointestinal tract. In gastroparesis the stomach and upper small intestine stands still and prevent digestion of diet as well as effects of orally administered drugs as the absorption is seriously reduced.

In another aspect, the compressed pharmaceutical dosage form comprises or consists of the active pharmaceutical ingredient melatonin for use in prevention, prophylaxis or treatment of a disease, such as insomnia disorders.

The invention also relates to a method of preparing the dosage form as defined above, comprising providing the active pharmaceutical ingredient, porous particles, binder, disintegrant, glidant, adhesion modifier and optionally taste masking agent and optionally pH modifier and optionally permeation enhancer and dry mixing the particles prior to direct compression of the particle mixture.

Alternatively, the active pharmaceutical ingredient may be loaded into the porous particles and the particles mixed with binder, disintegrant, glidant, adhesion modifier and optionally taste masking agent and optionally pH modifier and optionally permeation enhancer and dry mixing the particles prior to direct compression of the particle mixture.

### Brief description of the drawings

The invention will now be explained more closely by the description of different embodiments of the invention figures 1 to 10.
Fig. 1 illustrates capillary forces arising within and between particles of the dosage form of the invention.
Fig 2. Illustrates absorption of the active pharmaceutical ingredient across the oral mucus barrier localized under the tongue.
Fig. 3 shows thermogravimetric analysis (TGA) of metoclopramide (MTC) free drug and MTC loaded into porous particles (NLAB Silica 13) at around 30 wt% loading. The derivative curve shows the rate of weight loss of MTC as a function of temperature.
Fig. 4a,4b shows the differential scanning calorimetry analysis (DSC) of metoclopramide (MTC) as free drug and MTC loaded into porous silica particles (NLAB Silica 13) respectively, indicating MTC is in crystalline form before loading, and it is confined to an amorphous state after loading into porous particles.
Fig. 5 shows the nitrogen adsorption-desorption isotherm of a typical porous silica particle, indicating the particles are highly porous.
Fig. 6 shows the release kinetics of melatonin (MEL) loaded into porous silica particles (NLAB Silica) at 20 wt% loading compared to free melatonin.
Fig. 7 shows release kinetics of MEL from tablets manufactured via direct compression comprising MEL loaded into porous silica particles compared to a commercial sublingual tablet of MEL
Fig. 8 shows permeation of MTC across polyethersulfone hydrophilic membrane in Franz cell in PBS buffer released from tablets manufactured via direct compression comprising MTC mixed either with porous silica particles or non porous silica particles or tablets without silica particles.
Fig 9 shows release kinetics of MTC from tablets comprising MTC mixed with calcined porous silica particles, mixed with non porous silica particles, compressed without silica particles, MTC loaded into calcined porous silica particles and MEL loaded into porous silica particles that were calcined and subsequently chemically etched.
Fig 10 shows release kinetics of MTC from tablets comprising MTC loaded into calcined porous silica particles and MEL loaded into porous silica particles that were calcined and subsequently chemically etched.

### Detailed description of preferred embodiments of the invention

### Definitions and measurement methods

As used herein, a "active pharmaceutical ingredient" (API) means a pharmaceutical active compound having a preventive, prophylaxis or treating effect in/on a mammal body, such as a human. An active pharmaceutical ingredient may be a biological active compound, such as a low molecular substance a protein, peptide, antibody and the like.

Hardness of the dosage form can be measured using a Schleuniger type hardness tester or a Monsanto tester. The Monsanto design consists of "a barrel containing a compressible spring held between 2 plungers". The tablet is placed on the lower plunger, and the upper plunger is lowered onto it. In the Schleuniger design, am electric motor drives an anvil to compress a tablet at a constant rate. The tablet is pushed against a stationary anvil until it fractures. The hardness is preferably more than 10 N, or between 10 and 50 (or 1.0 and 5.0 kg/cm²). The hardness of the dosage form is at least 10 N and below 30 N as measured by a Schleuniger type hardness tester. The hardness may be between 10 and 30 N, or between 15 and 29 N, or between 20 and 29 N.

The friability value may be less than 2% or less than 1%.

As used herein, "substantially spherical" means that at least 90% or 95% of the particles are spherical.

As used herein, "instant prevention, prophylaxis or treatment of a disease" means that the active pharmaceutical ingredient has a pharmacological/medical effect in the patient's body within 30 minutes. A patient is a mammal, such as a human or an animal, such as a dog, cat, ape, cow or horse.

As used herein, "disintegration rate" means a falling apart of the tablet into pieces having a diameter of 2 mm or less. An example of a suitable test of determining disintegration rate is disclosed in AAPS PharmSciTech. 2011 Jun; 12(2): 544-552, Published online 2011 Apr 27, or in WO2013041851.

Porosity arises from both interstitial space between the excipients and internal pores of porous particles. Mercury (Hg) intrusion is a standard experimental method for determining porosity. In this method mercury is pushed into pores under applied pressure. However, there is a lower pore size limit of around 3.2 nm, below which, the mercury cannot penetrate. For porous materials with pore sizes in the mesoscale range of 1 to 50nm, nitrogen (N₂) sorption is commonly used to estimate pore size and pore volumes. The nitrogen sorption technique measures the available surface area of the porous materials. An empirical model is used to calculate the pore volume and pore size. The BET (Brunauer-Emmett-Teller) and BJH (Barrett, Joyner and Halenda) models are used to calculate porosity for the pores of silica particles.

In the disclosure, the full size-range of porosity is not accessible by a single analytical technique.

Scanning electron microscope (SEM) can be used to provide images of the porous particles. The diameter of the particles can be determined using SEM.

Porous particles here refer to porous silicon dioxide, porous titanium dioxide, porous magnesium dioxide, porous calcium carbonate, porous iron oxide, porous polymer, porous proteins, porous peptides, porous clays, porous minerals, metal organic frameworks, porous magnesium carbonate etc.

The present disclosure relates to a compressed pharmaceutical dosage form comprising an active pharmaceutical ingredient, porous particles and, optionally one or more pharmaceutically acceptable excipients. The porous particles are preferably silica particles. The silica particles may be calcinated.

The active pharmaceutical ingredients may be of classes I, II, III or IV of the Biopharmaceutics Classification System (BCS) (Amidon GL et al from 1995). In BCS an active pharmaceutical ingredient is considered highly soluble when the highest dose strength is soluble in 250 ml or less of aqueous media over the pH range of 1 to 7.5. Moreover, in BCS, an immediate release product is considered rapidly dissolving when no less than 85% of the labelled amount of the active pharmaceutical ingredient substance dissolves within 30 minutes using USP Dissolution Apparatus 1 at 100 rpm or Apparatus 2 at 50 rpm in a volume of 900 ml or less in the following media: 0.1 N HCI or simulated gastric fluid or pH 4.5 buffer, and pH 6.8 buffer or simulated intestinal fluid.

The active pharmaceutical ingredient may have a water solubility at 25 °C of 10 mg/ml or more according to Ph.Eur 9th Ed.

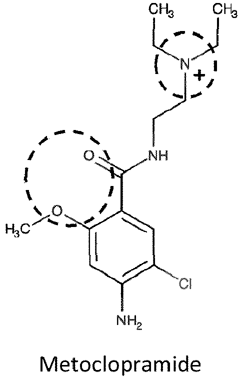

Metoclopramide is a medication used mostly for treatments of conditions in the esophagus, stomach and/or intestine. It is commonly used to treat and prevent nausea and vomiting, to enhance the gastric emptying rate in people with delayed stomach emptying, and to treat patients with gastroesophageal reflux disease. It is also used to treat migraine headaches. It is marketed under the tradenames Primperan^{™} and Reglan^{™}.

Oral administration and absorption from the gastrointestinal tract is the most common administration route for chronic medical treatment. However, intestinal absorption and bioavailability of metoclopramide is severely compromised by stomach malfunction and therefore alternative administration route are highly relevant to consider and develop. Metoclopramide first passage metabolism that may contribute to the variability.

Common side effects of metoclopramide include: feeling tired, diarrhea, and feeling restless. More serious side effects include: movement disorder like tardive dyskinesia, a condition called neuroleptic malignant syndrome, and depression.

Melatonin is a hormone that is produced by the pineal gland in animals and regulates sleep and wakefulness. It is sold over the counter for the treatment of insomnia in the United States, Canada and some European countries. The bioavailability of melatonin is reduced significantly through oral administration due to first pass effect. There are a number of sublingual tablet available on the market. Melatonin is a lipophilic compound with limited aqueous solubility, making it a good candidate as a model drug for use in combination with the dosage form of the invention. Its low dose also makes it feasible to fit in a sublingual tablet. Patent US 9,381,190 discloses use of silica as an absorbent to absorb dissolved melatonin in polyethylene glycol.

Other examples of suitable active pharmaceutical ingredients to be used in this mesoporous silica particles based FDT may be loratadine, donepezil, diphenhydramine pseudophedrine, lansoprazole, mirtazapine, rizatriptan, ondansetron, zolmitriptan, olanzapine and morphine.

Other suitable active pharmaceutical ingredients include erythromycin and domperidone, nitroglycerine and omeprazole.

Other suitable drugs include the phosphodiesterase type 5 (PDE5) inhibitor avanafil, lodenafil, mirodenafil, sildenafil, tadalafil, vardenafil, udenafil, zaprinast, benzamidenafil and dasantafil. Other suitable active pharmaceutical ingredients include nitrogycerine, adrenaline, oestrogen, progesterone and other hormones, peptides and proteins.

The compressed pharmaceutical dosage form may be used in prevention, prophylaxis or treatment of a disease. The disease can be any disease and the present invention is not limited to any particular disease. Indications of interest may be any disease, which causes nausea and/or vomiting. Anti-nausea active pharmaceutical ingredients and/or anti-vomiting active pharmaceutical ingredients may be used in the dosage form of this disclosure. Active pharmaceutical ingredients used in the treatment of cancer can be used because nausea is often caused by chemotherapy. Other indications are diseases related to the intragastrical tract or stomach, such as ulcers, spams and gastroparesis including diabetic gastroparesis. Other diseases may be heart failures, epilepsy or anaphylactic shock.

The porous particles may be manufactured using a sol-gel method such as the Stöber process, or a spray drying method.

Porous particles may be synthesized by cooperative self-assembly of silica species and organic templates such as cationic surfactants such as alkyltrimethylammonium templates with varying carbon chain lengths, and counterions such as cetyltrimethylammonium chloride (CTA+Cl- or CTAC) or cetyltrimethylammonium bromide (CTA+Br- or CTAB) or non-ionic species such as diblock and triblock polymer species such as copolymers of Polyethylene Oxide and Polypropylene Oxide for example Pluronic 123 surfactant. The formation of mesoporous silica particles occurs following the hydrolysis and condensation of silica precursor which can include alkylsilicates such as tetraethylorthosilcate TEOS or teramethylorthosilicate TMOS in solution or sodium silicate solution. The mesoporous silica particle size can be controlled by adding suitable additive agents e.g., inorganic bases, alcohols including methanol, ethanol, propanol and organic solvents such as acetone which affect the hydrolysis and condensation of silica species. The pore size can be influenced by hydrothermal treatment of the reaction mixture such as heating up to 100°C or even above and also with the additional of swelling agents in the form of organic oils and liquids that expand the surfactant micelle template. After condensation of the silica matrix, the templating surfactant can be removed by calcination typically at temperatures from 500°C up to 650°C for several hours which burns away the organic template resulting in a porous matrix of silica. The template may alternatively be removed by extraction and washing with suitable solvents such as organic solvents or acidic of basic solutions.

The use of surfactant templating methods has been expanded to create porous materials from other oxides including transition metals, such as titanium, niobium, tungsten, vanadium.

The porous silica particles may be manufactured by a sol-gel method comprising a condensation reaction of a silica precursor solution, such as sodium silicate or an aqueous suspension of silica nanoparticles as an emulsion with a non-miscible organic solution, oil, or liquid polymer in which droplets are formed by for example stirring or spraying the solution followed by gelation of the silica by means of change in pH and or evaporation of the aqueous phase. The porosity of the particles here are formed either by exclusion due to the presence of the non-miscible secondary phase or by the jamming of the silica nanoparticles during evaporation. The particles may further be treated by heating to induce condensation of the silica matrix and washed to remove the non-miscible secondary phase. Furthermore, the particles may be treated by calcination to strengthen the silica matrix.

The particles may also be collected and used without calcining following washing, filtering and drying

The porous particles may be manufactured as porous glass through a process of phase separation in borosilicate glasses (such as SiO₂-B₂O₃-Na₂O), followed by liquid extraction of one of the formed phases through the sol-gel process; or simply by sintering glass powder. During a thermal treatment, typically between 500°C and 760°C an interpenetration structure is generated, which results from a spinodal decomposition of the sodium-rich borate phase and the silica phase.

The porous particles may be manufactured using fumed process. In this method, fumed silica is produced by burning silicon tetrachloride in an oxygen-hydrogen flame producing microscopic droplets of molten silica, which fuses into amorphous silica particles in three-dimensional secondary particles, that then agglomerates into tertiary particles. The resulting powder has an extremely low bulk density and high surface.

The total amount of active pharmaceutical ingredient in the dosage form may be between 1 and 100 mg, or between 1 and 25 mg, or between 5 and 25 mg, or between 5 and 15 mg, or between 7 and 12 mg or about 10 mg. The concentration of the active pharmaceutical ingredient is between 5 and 15%, or between 6 and 13% w/w based on the total weight of the dosage form.

The porous particles may have a surface area between 100 and 1000 m²/g, or 200 and 800 m²/g, 300 and 1000 m²/g. The porous particles may have a pore volume between 0.2 and 3 cm³/g, or between 0.5 and 2.5 cm³/g. The porous particles may have an average pore size between 2 and 250 nm, or between 2 and 150 nm, or between 2 and 100 nm, or between 2 and 50 nm.

The porous particles may be nanoporous or mesoporous, or a mixture thereof. The diameter of the particles may be between 1 and 200 µm, or between 1 and 100 µm, or between 1 and 50 µm, or between 1 and 40 µm, or between 1 and 30 µm, or between 1 and 26 µm.

The concentration of the porous silica particles is 5% w/w or more, or 15%, 20%, 25%, 30%, 40%, 50%, 75%, 85%, 90% w/w based on the total weight of the dosage form. The concentration of the porous silica particles is between 14 and 24% w/w.

Optionally, pharmaceutical acceptable excipients may be added to the dosage form. Suitable pharmaceutical excipients (also called carriers, diluents and additives) may be found in "Pharmaceuticals - The Science of Dosage Form Designs", M. E. Aulton, Churchill Livingstone, 1988. Examples of excipients may be microcrystalline cellulose, such as PH-101, hydroxypropyl cellulose, such as low-substituted hydroxypropyl cellulose, saccharides, such as mannitol, stearates, such as magnesium stearate and cyclodextrins.

The one or more pharmaceutically acceptable excipient may be a binder selected from the group comprising or consisting of cellulose, starch and sugars. The binder may be microcrystalline cellulose.

The one or more pharmaceutically acceptable excipient may be a disintegrant selected from the group comprising or consisting of hydroxypropylmethylcellulose, cross-linked sodium carboxymethylcellulose, crosscarmellose, crosspovidone, sodium starch glycolate. and the disintegrant may be low-substituted hydroxypropylcellulose.

The one or more pharmaceutically acceptable excipient may be a binder, a disintegrant, a filler and a glidant, whereby the binder(s) and disintegrant(s) may be selected from as defined above.

The one or more pharmaceutically acceptable excipient may be a filler selected from the group comprising or consisting of mannitol, lactose, sucrose, magnesium stearate, glucose, plant cellulose and calcium carbonate. In one aspect, the filler is mannitol and/or magnesium stearate. The glidant may be magnesium stearate.

A ratio of porous particles:binder:disintegrant is between 3:10:1 and 2:8:1. The ratio may be 2.3:9:1. A ratio of binder:disintegrant is between 10:1 and 8:1.

The active pharmaceutical ingredient may be in admixture with the porous particles and/or the active pharmaceutical ingredient may be loaded in and/or loaded on in the porous particles (together referred to as loaded). A ratio of unloaded versus loaded ingredient is between 100:1 and 1:100, 50:1 and 1:50. The ratio may be between 2:1 and 6:1. For hydrophobic active pharmaceutical ingredients, the ratio may be between 100:1 and 50:50. For hydrophilic active pharmaceutical ingredients, the ratio may be between 50:1 and 10:1. This product based on mesoporous silica particle based dosage form may be administered to the patients when needed, or once or twice daily. The process of loading of an active pharmaceutical ingredient into the pores or the porous material includes dissolution of the active pharmaceutical ingredient in a suitable solvent and pouring/spraying or otherwise contacting the dissolved active pharmaceutical ingredient on the porous material. After drying, the active pharmaceutical ingredient is present in the porous material in amorphous form, which has an increased water solubility compared with the crystalline form of the active pharmaceutical ingredient substance.

The porosity of the dosage form is at least 5% or 10%, or 15% and at the most less than 30%, as measured by Hg intrusion and/or N₂ sorption. The porosity of the dosage form includes macro-pores, micro-pores, meso-pores and nano-pores. The ratio of intra-porosity versus inter-porosity is between 1:2 and 20:1, or between 0.5:1 and 6:1. The ratio may be 2:1.

The particles may have different forms and shapes, such as spherical or rod-like. In one dosage form, particles having one spherical shape may be used, or a mixture of particles having different shapes may be used.

The invention relates to a compressed pharmaceutical dosage form comprising or consisting of
one or more active pharmaceutical ingredients present at a concentration between 7 and 12 % w/w based on the total weight of the dosage form
porous calcinated and spherical particles, wherein the porous particles have a diameter between 1 and 50 µm, as measured by SEM and the particles are present at a concentration between 14 and 24% w/w based on the total weight of the dosage form and,
wherein the porous particles are silica mesoporous particles having a surface area between 200 and 800 m²/g and/or a pore volume between 0.6 and 2 cm³/g and/or an average pore size between 2 and 30 nm,
a binder selected from the group comprising cellulose or starch or microcrystalline cellulose, a disintegrant selected from the group comprising hydroxypropylmethylcellulose, cross-linked sodium carboxymethylcellulose, crosscarmellose, crosspovidone, sodium starch glycolate or hydroxypropylmethylcellulose, whereby a ratio of porous particles:binder:disintegrant is between 3:10:1 and 2:8:1,
whereby the one or more active pharmaceutical ingredients is in admixture with and/or loaded into said particles, whereby a ratio of unloaded versus loaded ingredient is between 100:1 and 1:100, and
whereby a porosity of the dosage form is more than 5% and less than 30%, as measured by Hg intrusion and/or N₂ sorption, and
the hardness of the dosage form is more than 10 N and less than 29 N using a Schleuniger type tester.

The friability value is preferably around 1% or less.

In one aspect, the active pharmaceutical ingredient is metoclopramide and/ or melatonin. In another aspect, the particles are calcinated. In an aspect, the active pharmaceutical ingredient is in admixture with or loaded into said particles. In an aspect, the particles are substantially spherical. Mannitol may be used as a filler. magnesium stearate may be used as a glidant. The dosage form may be an immediate release product for acute or instant prevention, prophylaxis or treatment of a disease/disorder/ condition. The disintegration rate may be between 0.25 and 5 minutes, or between 0.25 and 1 minute. Or the disintegration rate may be at most 30 seconds, or 20 seconds, as measured by USP disintegration apparatus.

The dosage form may have a length of 2 cm at the most, preferably 1 cm and a width of 2, or 1 cm at the most. The thickness may be between 0.1 and 15 mm, or between 0.2 and 10 mm, or between 0.2 and 0.755 mm, or between 0.2 and 0.6 mm.

At least a portion of the total porous particles may be coated. The coating may be a taste-masking coating or an mucoadhesive coating. The coating may also be used to improve hydrophilicity or lipophilicity. The coating may even be used to charge the particles' surface. Suitable coating agents may be selected from the group comprising or consist of amino acids, surfactant, polymer, lipids, cellulose or derivatives thereof, saccharides, stearates like magnesium stearate, peptides, proteins and nanoparticles, which may be porous.

Examples of amino acids may be L-lysine, L-alanine, glycine and L-tyrosine.

### Examples

### Example 1

Porous particles were manufactured using a sol gel reaction comprising an emulsion process containing an aqueous silica solution and a non-miscible oil and subsequent condensation of the silica via control of the solution pH and temperature. The porosity of the particles was controlled by the condensation rate of the silica. Then the samples were filtered from oil phase, washed and calcined at 650°C for 6 hours. Various particles were produced via various in reaction conditions generating particles with different particle size, pore size and pore volumes designated Samples 1-5 in table 1.

**Table 1: Bulk density and pore properties of a range of prototype porous silica particles.**

| | **Bulk density (g/ml)** | **Surface area (m2/g)** | **Pore volume (cm3/g)** | **average pore size (nm) 15** |
|---|---|---|---|---|
| **Sample 1** | 0.46 | 728 | 0.95 | 5.3 |
| **Sample 2** | 0.2 | 804 | 1.86 | 9.3 |
| **Sample 3** | 0.34 | 327 | 0.85 | 11.8 |
| **Sample 4** | 0.22 | 287 | 1.83 | 31.5 |
| **Sample 5** | 0.17 | 277 | 1.56 | 28.4 |

Porous particles in this invention refers to silica with pore size between 2 nm and 200 nm from BET. Porous particles in this invention refers to silica pore volume between 0.2 cm³/g and 2.5 cm³/g from BET.

In order to evaluate the porosity of the tablets over the full size-range of 0.2 nm to 500 µm both methods, Hg intrusion and N₂ sorption, have been used. The contribution to the porosity for the pores in the meso-size rage was calculated from the determined porosity of the porous silica particles by themselves before mixing with the other excipients. The overall porosity of the tablets exceeded 40%.

**Table 2: Porosity of porous prototype tablets.**

| Porosity (%) contribution from pore size ≥3.2nm^{a} | Porosity (%) contribution from pore size ≤3nm^{b} | Total porosity (%) 30 |
|---|---|---|
| 37.7% | ≥ 9 % | ≥ 40 % |

| | | |
|---|---|---|
| ^{a}:Porosity measured by Hg intrusion method. ^{b}: Porosity calculated by N₂ sorption method. | | |

### Example 2

Loading method: metoclopraminde (MTC) was dissolved in ethanol (analytical grade) at a concentration of 20 mg/ml, followed by addition of porous silica particles (NLAB-Silica 13). The porous silica particles are characterized by a surface area of around 330 m²/g and/or a pore volume around 0.8 cm³/g and/or an average pore size 12 nm, spherical with average diameter of 10 micrometers. Similar silica particles (NLAB-Silica 13 or NLAB-Silica^{™}) were used in all experiments loading MTC.

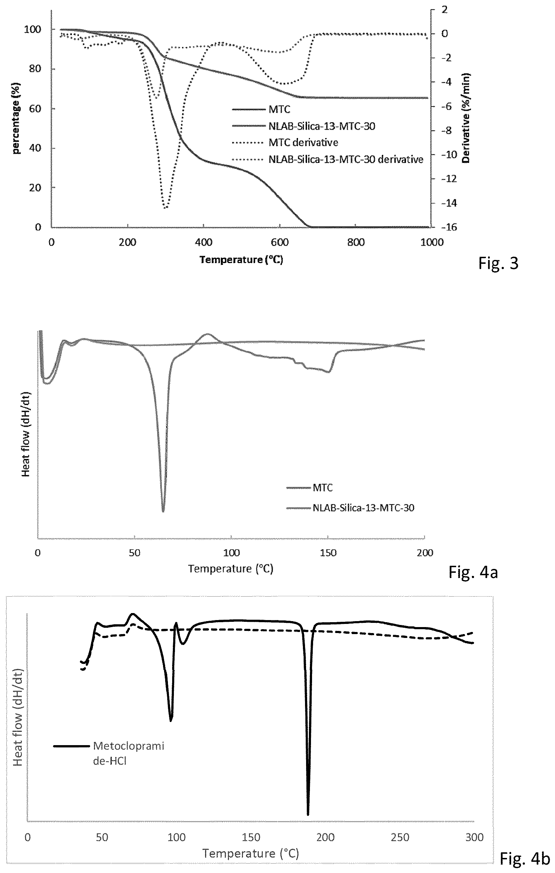

After stirring for 10 minutes, the solvent was removed by evaporation on a rotary evaporator. The dry powder was collected and dried in an oven under vacuum overnight at 30°C. The samples were characterized by thermogravimetric analysis (TGA) and differential scanning calorimetry analysis (DSC) as shown figure 3 and 4a (MTC base), 4b (MTC salt).

### Example 3

Method for manufacturing of a tablet.

The ingredients as listed in table 3 were mixed and compressed into a tablet at 10kN using a manual tablet press.

**Table 3. Formulation of metoclopramide sublingual tablets.**

| **Ingredients** | **Formulation 1** | **Formulation 2** | **Formulation 3** | **Formulation 4** |
|---|---|---|---|---|
| **Metoclopramide HCI** | **10** | **10** | **10** | |
| **Nanoporous silica** | **21.5** | **-** | **-** | **-** |
| **Non-porous silica nanoporous silica loaded with metoclopramide (30 wt%)** | **-** | **21.5** | **-** | **-** |
| | **-** | **-** | **-** | **33.33** |
| **Microcrystalline cellulose (PH-101)** | **81** | **81** | **81** | **81** |
| **Low-substitute** | | | | |
| **Hydroxypropyl** | **9** | **9** | **9** | **9** |
| **Cellulose** | | | | |
| **Mannitol** | **27.5** | **27.5** | **49.25** | **27.5** |
| **Magnesium stearate** | **0.75** | **0.75** | **0.75** | **0.75** |
| **Total tablet weight (mg)** | **150** | **150** | **150** | **150** |

### Example 4

Tablet manufactured with direct compression were placed on top of a Porosity 3-25mm diameter filter in a glass funnel, followed by addition of 10 ml water on to the tablet. The tablet disintegrated, and water dissolved the drug, which than passed across the filter. The filtrate was collected at different time points and UV absorbances were used to quantify the concentration of the drug in the filtrate.

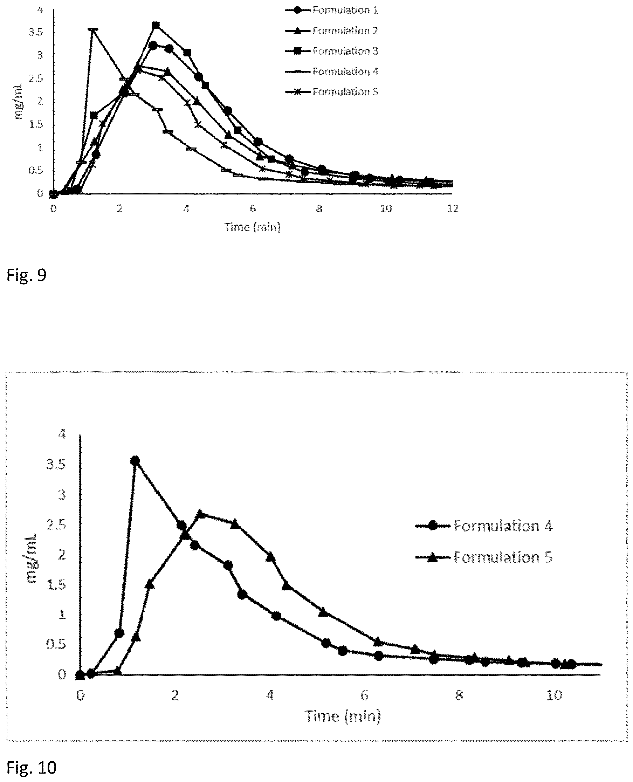

Figure 9 shows the drug concentration-time profiles in the filtrate from three formulations 1,2,3 mentioned in table 3. The concentration was calculated from the UV absorbance of metoclopramide solution at 308 nm. Formulation 1 and 4, with the presence of silica (mixing or loaded with metoclopramide), showed faster release than formulation 3 in which silica is absent. The results reflect the release of metoclopramide in small volume of liquid during static condition.

### Example 5

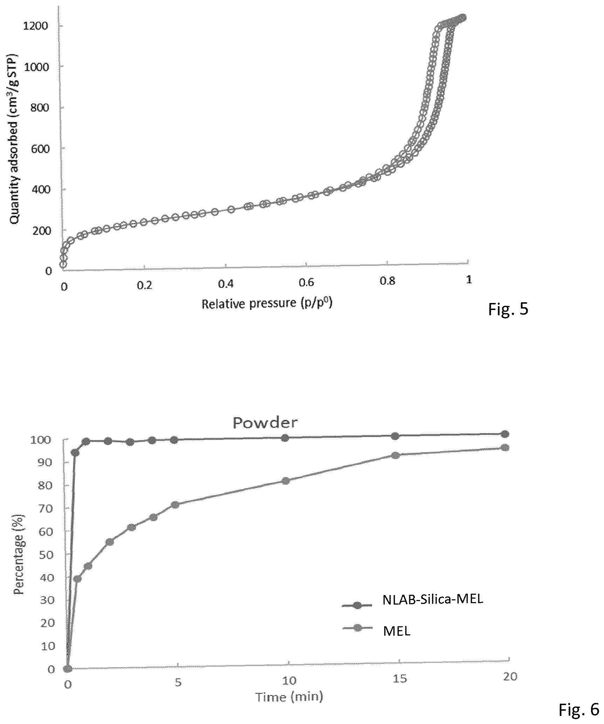

Figure 5 shows an N₂ adsorption-desorption isotherm of samples 2 in table 1. The samples have both high surface area up to 800 m²/g and high pore volume up to 1.8 cm³/g. Figure 5 shows that the porous silica particle is highly porous.

### Example 6

A range of different NLAB-Silica^{™} particles, having different porous particle characteristics, were investigated for the loading of melatonin (MEL). The key parameters guiding the choice of carrier were the dissolution kinetics of the loaded drug and the loading amount. NLAB-Silica particles were found be a suitable carrier for the enhancement of release kinetics of MEL. The loading of melatonin into the silica materials was performed using the evaporation method. This involves combining a concentrated solution of MEL with the silica particles, then removing the solvent and/or drying the sample prior to further processing. Ethanol was used as a solvent for loading melatonin in NLAB-Silica^{™}.

High performance liquid chromatography (HPLC) was applied to determine the free melatonin when mixed with loaded particles, and the data indicate stability (i.e. no degradation) during loading as well as after storage at 4°C. The drug content of the melatonin-loaded silica materials was determined using Thermogravimetric Analysis (TGA), The loading amount is referred to as a weight percentage of the MEL compared to the combined weight of NLAB-Silica^{™} and drug.

The physical form of the loaded melatonin was determined through Differential Scanning Calorimetry (DSC).

The release kinetics of melatonin was monitored using a USP Apparatus 2 dissolution testing system. The release was performed in 500 ml buffer at pH 6.8 (reflecting the pH in the oral cavity, typically in the range of pH 6.7 to 7.3) at 50 rpm stirring rate and 37°C. The dose in the dissolution tests was equivalent to 5 mg of melatonin. Melatonin was bought from PureBulk, Inc. USA. The dosage form with melatonin was compared to reference product from CVS Health, USA using the technology mentioned in US 9,381,190 Other ingredients in the formulation include Microcrystalline cellulose (MCC) and Low-Substituted Hydroxypropyl Cellulose (L-HPC) Mannitol and magnesium stearate.

The drug loading of 20 wt% in one type of NLAB Silica particles was obtained with the optimal dissolution kinetics. Melatonin loaded into NLAB Silica^{™} greatly improved the dissolution kinetics, which reached 99% within 1 minute, compared to the free melatonin reaching 93% after 20 minutes. The dosage form with melatonin in NLAB Silica^{™} was developed and an optimized formulation showed enhanced dissolution kinetics, which out-performed the commercial formulation.

Figure 6 shows that the dissolution rate was significantly increased for the loaded sample compared to the free MEL. The dissolution reaches 100% after 1 minute at a low stirring rate of 50 rpm, which is an indication that melatonin is very easy to dissolve under neutral pH after loading.

Two pharmaceutical compositions were compared to find a better-performed formulation (Table 4). The difference between the two pharmaceutical compositions is regarding the amount of MCC (binder), and tablet size. In pharmaceutical compositions 2, more loaded sample was added to take the weight loss of silanol groups in silica particles into consideration. With low amount of binder (20%), the powder can still be compressed successfully at moderate pressure.

**Table 4. Formulation composition of the final formulation.**

| | Recipe 1 | | Recipe 2 | |
|---|---|---|---|---|
| | Tablet weight (mg) | 150.25 | Tablet weight (mg) | 120 |
| **Ingredients** | **Percentage(%)** | **Amount (mg)** | **Percentage(%)** | **Amount (mg)** |
| NLAB-Silica-18-MEL-20 | 16.6 | 24.9 | 23.5 | 28.2 |
| Mannitol | 52.9 | 79.5 | 51 | 61.2 |
| MCC PH101 | 25 | 37.6 | 20 | 24 |
| L-HPC | 5 | 7.5 | 5 | 6 |
| Magnesium stearate | 0.5 | 0.75 | 0.5 | 0.6 |
| SUM | 100 | 150.25 | 100 | 120 |

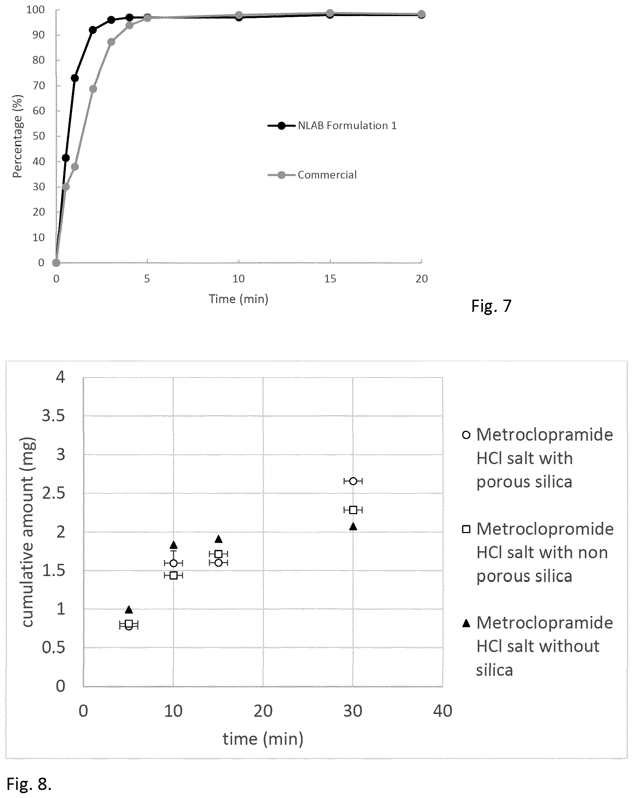

Experiments were conducted using the same ratio of excipients as pharmaceutical compositions 1, but melatonin was mechanically mixed with silica particles rather than loaded into the particles. Tablets of the mixed ingredients showed good dissolution kinetics of MEL The kinetic release of MEL from a tablet formulation based on NLAB silica loaded MEL was compared with a commercially available tablet (i.e. reference) as shown in Figure 7. The tablet NLAB silica-MEL (pharmaceutical compositions 1) showed the fastest dissolution, which is clearly more rapid than the commercially available tablet product. The supplier of the commercial tablet product (CVS Health Rapid Absorption Melatonin) claims that melatonin is in dissolved state in their tablet. Importantly, the results of the release rate indicate that NLAB formulation, where melatonin is in solid dispersion form, is superior to the product where melatonin is in dissolved form.

This study demonstrated that loading melatonin into NLAB SilicaTM particles yields enhanced release kinetics and higher apparent solubility, as well as better handability of the free drug. The loading was performed by adding silica particles to melatonin solution in ethanol (analytical grade) and removing the solvent by evaporation. The release kinetics showed immediate release profile of melatonin, which was dramatically better than release of the free drug. NLAB Silica formulation of MEL was developed using different pharmaceutical excipients, which outperformed the commercial tablet product, with more rapid release kinetics at pH 6.8.

### Example 7

Tablets comprising MTC were manufactured by direct compression according to the pharmaceutical ingredients listed in composition in Table 5. They were placed in Franz cell above a polyethersulfone hydrophilic membrane (millipore), and the donor compartment contained PBS (1 ml, pH 6.8), the acceptor compartment contained PBS (6 ml, pH 7.4) at a controlled temperature at 37°C. The permeation of metoclopramide across the artificial membrane was determined through analysis of aliquots taken from the acceptor compartment at regular time intervals. In the acceptor samples were metoclopramide quantified by UV absorption against a standard calibration curve measured in the same buffer at 308nm. Even under static conditions the permeation of metoclopramide across the artificial membrane in the Franz cell was rapid. The results show that metoclopramide permeated with the similar kinetics for all formulations in the first 15 minutes and even showed superior permeation kinetics with the formulation containing the porous silica particles after 30 minutes with close to 90% of metroclopramide in the formulation permeating from the formulation containing porous particles compared to around 80 % and 65% of metrocropimide permeating from the tablets with non porous particles and without silica respectively. See figure 8.

The friability and hardness of the tablets are shown in table 6.

**Table 5.**

| Ingredients | Formulation 1 | Formulation 2 | Formulation 3 |
|---|---|---|---|
| Metoclopramide HCI | 10 | 10 | 10 |
| Nanoporous silica | 21.5 | - | - |
| Non-porous silica | - | 21.5 | - |
| Microcrystalline cellulose (PH-101) | 81 | 81 | 81 |
| Low-su bstitute Hydroxypropyl Cellulose | 9 | 9 | 9 |
| Mannitol | 27.75 | 27.75 | 49.25 |
| Magnesium stearate | 0.75 | 0.75 | 0.75 |
| Total tablet weight (mg) | 150 | 150 | 150 |

**Table 6**

| Code | Friability % | Hardness (N) |
|---|---|---|
| | mean (n=3) | |
| Formulation 1 | 1.95 ± 0.14 | 25.6 ± 3.81 |
| Formulation 2 | 2.54 ±0.27 | 19.33 ± 2.91 |
| Formulation 3 | 1.58 ±0.46 | 19.96 ± 3.51 |

### Example 8

Five different formulations were tested;
Formulation 1 metoclopramide mixed with calcined porous silica,
Formulation 2 metoclopramide mixed with non porous silica,
Formulation 3 metoclopramide tableted without silica,
Formulation 4 metoclopramide loaded into calcined porous silica, and
Formulation 5 metoclopramide loaded into calcined and etched porous silica.

The particles were manufactured as described above and loaded as described in example 2. The friability and hardness of the tablets are shown in table 6.

**Table 7**

| **Ingredients** | **Formulation 1** | **Formulation 2** | **Formulation 3** | **Formulation 4** | **Formulation 5** |
|---|---|---|---|---|---|
| Metoclopramide Hcl | 10 | 10 | 10 | | - |
| Nanoporous silica | 21.5 | - | - | - | - |
| Non-porous silica nanoporous silica loaded with metoclopramide (30 wt%) | | 21.5 | | | |
| | - | - | - | 33.33 | 33.33 |
| Microcrystalline cellulose (PH-101) | 81 | 81 | 81 | 81 | 81 |
| Low-substitute Hydroxypropyl Cellulose | 9 | 9 | 9 | 9 | 9 |
| Mannitol | 27.5 | 27.5 | 49.25 | 27.5 | 27.75 |
| Magnesium stearate | 0.75 | 0.75 | 0.75 | 0.75 | 0.75 |
| **Total tablet weight (mg)** | 150 | 150 | 150 | 150 | 150 |

**Table 8**

| Code | Friability % | Hardness (N) |
|---|---|---|
| | mean (n=3) | |
| Formulation 1 | 1.95 ± 0.14 | 25.6 ± 3.81 |
| Formulation 2 | 2.54 ± 0.27 | 19.33 ± 2.91 |
| Formulation 3 | 1.58 ± 0.46 | 19.96 ± 3.51 |
| Formulation 4 | 1.03 ± 0.26 | 29.43 ± 1.90 |

Dissolution studies in small volume.

Tablets or dosage forms manufactured via direct compression of powdered excipients were placed on top of a 25mm diameter filter (porosity 3) in a glass funnel, followed by addition of 10 ml Simulated Saliva Fluid (SSF) at pH 6.8 over to the dosage form. The dosage form disintegrated, and SSF dissolved the drug, which than passed through the filter. The filtrate was collected at different time points the concentration of the metoclopramide in each aliquot was measured by UV absorption at 272 nm.

Figure 9 shows that the dissolution kinetics were similar for the dosage forms produced by dry mixing of metoclopramide with standard excipients with porous and non porous silica as well as just the metoclopramide with mannitol replacing the silica. Surprisingly, the dissolution of metoclopramide from the dosage form comprising the drug loaded into calcined silica show a dramatic reduction in time to Cₘₐₓ. This indicates that the release out of the porous particles and through the dosage form was enhanced by formulation of metoclopramide into the calcined silica particles.

The release of API (metoclopramide)[a highly soluble drug] from a dosage form comprising the API loaded into [calcined] porous silica particles showed surprisingly quicker release profile compared to dosage forms comprising the API mixed with the same [calcined] porous silica particles, or the API loaded into particles that were chemically etched after calcination or dosage forms without silica particles. The more rapid release profile is highly desirable from a product perspective.

The release kinetics of metoclopramide loaded into calcined silica particles showed a Cₘₐₓ maximum released amount in 1 minute compared to 3 minutes for dosage forms with admixtures of API and particles and with API loaded into particles that were chemically etched (formulation 5).

A dosage form comprising metoclopramide loaded into (calcined) silica particles showed better friability compared to dosage forms comprising the mixed API and silica formulations.

### Example 9

Dissolution studies of dosage forms comprising metoclopramide loaded into calcined porous silica and loaded into calcined and etched porous silica.

The rate of metoclopramide concentration dissolving from the dosage forms comprising metoclopramide loaded into calcined porous silica was significantly higher than the formulation metoclopramide loaded into calcined and etched porous silica with Cₘₐₓ reached in 1 minute versus 3 minutes for the calcined and etched silica particles respectively.

The results indicate that the release of metoclopramide was enhanced in the case of using the hydrophobic, calcined silica compared to the etched hydrophilic silica.

The results are shown in figure 10.

The purpose of the invention is to increase the absorption of API into the oral mucosa by increasing the exposure of the API to the oral mucosa by the effective dispersion of particles through the oral mucosa. The addition of porous silica particles into the formulation yields dosage forms with desirable hardness and friability. The addition of mechanically robust silica particles in the size range 1-100 µm enhances the performance of the disintegrant compared to dosage forms without silica particles. It was observed that the dosage forms formulated with silica particles disintegrated surprisingly rapidly and more homogeneously compared to dosage forms without silica particles.

The API may be absorbed into the systemic system and or the lymphatic system.

By formulating API into porous silica is particularly beneficial for sublingual dosage forms to provide physical stability, protection, of the molecule (API). The disintegration property of the dosage forms is critical to ensure uniform disintegration to avoid variable dose.

If the dosage form breaks apart into pieces, the release of the API may be non-homogeneous and parts of the dosage form may be swallowed preventing absorption through the sublingual route.

The observed surprising increase in release rate of the API from the dosage form with the API loaded into (hydrophobic) silica is highly desirable from a product perspective.

In example YY, the use of calcined silica particles improved the release of a highly soluble API from a dosage form with desirable physical properties in terms of hardness and friability

## Claims

1. A compressed pharmaceutical dosage form comprising
one or more active pharmaceutical ingredients present at a concentration between 2 and 60 % w/w based on the total weight of the dosage form,
porous particles, wherein the porous particles have a diameter between 1 and 100 µm, as measured by SEM and the porous particles are silica mesoporous particles have a surface area between 200 and 1000 m²/g and/or a pore volume between 0.2 and 3 cm³/g and/or an average pore size between 2 and 250 nm and the particles are present at a concentration between 5 and 70% w/w based on the total weight of the dosage form and,
optionally one or more pharmaceutically acceptable excipients,
whereby the one or more active pharmaceutical ingredients is in admixture with and loaded into said particles, whereby a ratio of unloaded versus loaded ingredient is between 100:1 and 1:100, and
whereby a porosity of the dosage form is between 5 and 29% as measured by Hg intrusion and/or N₂ sorption,
a hardness of the dosage form is more than 5 N and less than 30 N using a Schleuniger type hardness tester, and
the friability value is preferably 2% or less.

2. The compressed pharmaceutical dosage form according to claim 1, comprising
one or more active pharmaceutical ingredients present at a concentration between 2 and 60 % w/w based on the total weight of the dosage form, whereby the active pharmaceutical ingredients has a water solubility at 25 °C of 10 mg/ml or more according to Ph.Eur 9^{th} Ed.

3. The compressed pharmaceutical dosage form according to any one of claims 1 or 2, wherein ratio of unloaded versus loaded ingredient is for hydrophobic active pharmaceutical ingredients is between 100:1 and 50:50, and for hydrophilic active pharmaceutical ingredients between 50:1 and 10:1.

4. The compressed pharmaceutical dosage form according to any one of the preceding, wherein the friability value is around 1% or less.

5. The compressed pharmaceutical dosage form according to any one of the preceding claims, wherein the dosage form is a tablet with a hardness between 15 and 29 N.

6. The compressed pharmaceutical dosage form according to any one of the preceding claims, wherein the ratio of unloaded versus loaded ingredient is between 1:99 and 50:50.

7. The compressed pharmaceutical dosage form according to any one of the preceding claims, wherein a ratio of intra-porosity versus inter-porosity is between 1:2 and 20:1.

8. The compressed pharmaceutical dosage form according to any one of the preceding claims, comprising the one or more pharmaceutically acceptable excipient as a binder selected from the group comprising cellulose, microcrystalline cellulose, starch and polyvinyl pyrrolidone.

9. The compressed pharmaceutical dosage form according to any one of the preceding claims, comprising the one or more pharmaceutically acceptable excipient as a disintegrant selected from the group comprising hydroxypropylmethylcellulose, cross-linked sodium carboxymethylcellulose, crosscarmellose, crosspovidone, low-substituted hydroxypropylcellulose and sodium starch glycolate.

10. The compressed pharmaceutical dosage form according to any one of the preceding claims, wherein a ratio of binder:disintegrant is between 10:1 and 8:1.

11. The compressed pharmaceutical dosage form according to any one of the preceding claims, wherein the active pharmaceutical ingredient is metoclopramide or melatonin.

12. The compressed pharmaceutical dosage form according to any one of the preceding claims, wherein the active pharmaceutical ingredient is selected from the group comprising loratadine, donepezil, diphenhydramine, pseudoephedrine, lansoprazole, mirtazapine, rizatriptan, ondansetron, zolmitriptan, olanzapine, morphine, erythromycin,domperidone, omeprazole, avanafil, lodenafil, mirodenafil, sildenafil, tadalafil, vardenafil, udenafil, zaprinast, benzamidenafil, nitroglycerine, oestrogen, progesterone, adrenaline and dasantafil.

13. The compressed pharmaceutical dosage form according to any one of the preceding claims, wherein the active pharmaceutical ingredient is metoclopramide for use in prevention, prophylaxis or treatment of a disease, such as gastroparesis.

14. The compressed pharmaceutical dosage form according to any one of the preceding claims, wherein the active pharmaceutical ingredient is melatonin for use in prevention, prophylaxis or treatment of a disease, such as insomnia disorders.

## Patentansprüche

1. Komprimierte pharmazeutische Dosierungsform, umfassend
einen oder mehrere pharmazeutische Wirkstoffe, die in einer Konzentration zwischen 2 und 60 Gew.-%, bezogen auf das Gesamtgewicht der Dosierungsform, vorhanden sind,
poröse Teilchen, wobei die porösen Teilchen einen Durchmesser zwischen 1 und 100 µm aufweisen, gemessen durch SEM, und die porösen Teilchen mesoporöse Siliziumdioxidteilchen sind, die einen Oberflächenbereich zwischen 200 und 1000 m²/g und/oder ein Porenvolumen zwischen 0,2 und 3 cm³/g und/oder eine durchschnittliche Porengröße zwischen 2 und 250 nm aufweisen, und die Teilchen in einer Konzentration zwischen 5 und 70 Gew.-%, bezogen auf das Gesamtgewicht der Dosierungsform, vorhanden sind, und wahlweise einen oder mehrere pharmazeutisch verträgliche Hilfsstoffe,
wobei der eine oder die mehreren pharmazeutischen Wirkstoffe in Mischung mit den Teilchen sind und in diese geladen sind, wobei ein Verhältnis von unbeladenem versus beladenem Inhaltsstoff zwischen 100 : 1 und 1 : 100 liegt, und
wobei eine Porosität der Dosierungsform zwischen 5 und 29 % liegt, gemessen durch Hg-Eindringen und/oder N₂-Sorption,
eine Härte der Dosierungsform mehr als 5 N und weniger als 30 N unter Verwendung eines Härteprüfers vom Schleuniger-Typ beträgt, und
der Brüchigkeitswert vorzugsweise 2 % oder weniger beträgt.

2. Komprimierte pharmazeutische Dosierungsform nach Anspruch 1, umfassend
einen oder mehrere pharmazeutische Wirkstoffe, die in einer Konzentration zwischen 2 und 60 Gew.-%, bezogen auf das Gesamtgewicht der Dosierungsform, vorhanden sind, wobei die pharmazeutischen Wirkstoffe eine Wasserlöslichkeit bei 25 °C von 10 mg/ml oder mehr gemäß Ph.Eur 9. Auflage aufweisen.

3. Komprimierte pharmazeutische Dosierungsform nach einem der Ansprüche 1 oder 2, wobei das Verhältnis von unbeladenem versus beladenem Inhaltsstoff für hydrophobe pharmazeutische Wirkstoffe zwischen 100 : 1 und 50 : 50 und für hydrophile pharmazeutische Wirkstoffe zwischen 50 : 1 und 10 : 1 liegt.

4. Komprimierte pharmazeutische Dosierungsform nach einem der vorstehenden,
wobei der Brüchigkeitswert ungefähr 1 % oder weniger beträgt.

5. Komprimierte pharmazeutische Dosierungsform nach einem der vorstehenden Ansprüche, wobei die Dosierungsform eine Tablette mit einer Härte zwischen 15 und 29 N ist.

6. Komprimierte pharmazeutische Dosierungsform nach einem der vorstehenden Ansprüche, wobei das Verhältnis von unbeladenem versus beladenem Inhaltsstoff zwischen 1 : 99 und 50 : 50 liegt.

7. Komprimierte pharmazeutische Dosierungsform nach einem der vorstehenden Ansprüche, wobei ein Verhältnis von Intraporosität versus der Interporosität zwischen 1 : 2 und 20 : 1 liegt.

8. Komprimierte pharmazeutische Dosierungsform nach einem der vorstehenden Ansprüche, umfassend den einen oder die mehreren pharmazeutisch verträglichen Hilfsstoffe als Bindemittel, ausgewählt aus der Gruppe umfassend Cellulose, mikrokristalline Cellulose, Stärke und Polyvinylpyrrolidon.

9. Komprimierte pharmazeutische Dosierungsform nach einem der vorstehenden Ansprüche, umfassend den einen oder die mehreren pharmazeutisch verträglichen Hilfsstoffe als Sprengmittel, ausgewählt aus der Gruppe, umfassend Hydroxypropylmethylcellulose, vernetzte Natriumcarboxymethylcellulose, Crosscarmellose, Crosspovidon, niedrigsubstituierte Hydroxypropylcellulose und Natriumstärkeglykolat.

10. Komprimierte pharmazeutische Dosierungsform nach einem der vorstehenden Ansprüche, wobei ein Verhältnis von Bindemittel : Sprengmittel zwischen 10 : 1 und 8 : 1 liegt.

11. Komprimierte pharmazeutische Dosierungsform nach einem der vorstehenden Ansprüche, wobei der pharmazeutische Wirkstoff Metoclopramid oder Melatonin ist.

12. Komprimierte pharmazeutische Dosierungsform nach einem der vorstehenden Ansprüche, wobei der pharmazeutische Wirkstoff ausgewählt ist aus der Gruppe umfassend Loratadin, Donepezil, Diphenhydramin, Pseudoephedrin, Lansoprazol, Mirtazapin, Rizatriptan, Ondansetron, Zolmitriptan, Olanzapin, Morphin, Erythromycin, Domperidon, Omeprazol, Avanafil, Lodenafil, Mirodenafil, Sildenafil, Tadalafil, Vardenafil, Udenafil, Zaprinast, Benzamidenafil, Nitroglycerin, Östrogen, Progesteron, Adrenalin und Dasantafil.

13. Komprimierte pharmazeutische Dosierungsform nach einem der vorstehenden Ansprüche, wobei der pharmazeutische Wirkstoff Metoclopramid zur Verwendung bei der Vorbeugung, Prophylaxe oder Behandlung einer Erkrankung, wie Gastroparese, ist.

14. Komprimierte pharmazeutische Dosierungsform nach einem der vorstehenden Ansprüche, wobei der pharmazeutische Wirkstoff Melatonin zur Verwendung bei der Vorbeugung, Prophylaxe oder Behandlung einer Erkrankung, wie Schlafstörungen, ist.

## Revendications

1. Forme galénique pharmaceutique comprimée comprenant
un ou plusieurs ingrédients pharmaceutiques actifs présents à une concentration comprise entre 2 et 60 % p/p sur la base du poids total de la forme galénique,
des particules poreuses, dans laquelle les particules poreuses ont un diamètre compris entre 1 et 100 µm, tel que mesuré par MEB et les particules poreuses sont des particules mésoporeuses de silice ont une superficie comprise entre 200 et 1000 m²/g et/ou un volume de pore compris entre 0,2 et 3 cm³/g et/ou une taille moyenne de pore comprise entre 2 et 250 nm et les particules sont présentes à une concentration comprise entre 5 et 70 % p/p sur la base du poids total de la forme galénique et, éventuellement un ou plusieurs excipients pharmaceutiquement acceptables,
moyennant quoi le ou les ingrédients pharmaceutiques actifs sont en mélange avec et chargés dans lesdites particules, moyennant quoi un rapport de l'ingrédient non chargé par rapport à l'ingrédient chargé est compris entre 100:1 et 1:100, et
moyennant quoi une porosité de la forme galénique est comprise entre 5 et 29 % telle que mesurée par intrusion de Hg et/ou sorption de N₂,
une dureté de la forme galénique est supérieure à 5 N et inférieure à 30 N en utilisant un testeur de dureté de type Schleuniger, et
la valeur de friabilité est de préférence de 2 % ou moins.

2. Forme galénique pharmaceutique comprimée selon la revendication 1, comprenant
un ou plusieurs ingrédients pharmaceutiques actifs présents à une concentration comprise entre 2 et 60 % p/p sur la base du poids total de la forme galénique, moyennant quoi les ingrédients pharmaceutiques actifs ont une solubilité dans l'eau à 25 °C de 10 mg/ml ou plus selon la Ph. Eur. 9^{e} Éd.

3. Forme galénique pharmaceutique comprimée selon l'une quelconque des revendications 1 ou 2, dans laquelle le rapport de l'ingrédient non chargé par rapport à l'ingrédient chargé est pour des ingrédients pharmaceutiques actifs hydrophobes est compris entre 100:1 et 50:50, et pour des ingrédients pharmaceutiques actifs hydrophiles compris entre 50:1 et 10:1.

4. Forme galénique pharmaceutique comprimée selon l'une quelconque des précédentes,
dans laquelle la valeur de friabilité est d'environ 1 % ou moins.

5. Forme galénique pharmaceutique comprimée selon l'une quelconque des revendications précédentes, dans laquelle la forme galénique est un comprimé ayant une dureté comprise entre 15 et 29 N.

6. Forme galénique pharmaceutique comprimée selon l'une quelconque des revendications précédentes, dans laquelle le rapport de l'ingrédient non chargé par rapport à l'ingrédient chargé est compris entre 1:99 et 50:50.

7. Forme galénique pharmaceutique comprimée selon l'une quelconque des revendications précédentes, dans laquelle un rapport de l'intra-porosité par rapport à l'inter-porosité est compris entre 1:2 et 20:1.

8. Forme galénique pharmaceutique comprimée selon l'une quelconque des revendications précédentes, comprenant le ou les excipients pharmaceutiquement acceptables comme liant choisi dans le groupe comprenant la cellulose, la cellulose microcristalline, l'amidon et la polyvinylpyrrolidone.

9. Forme galénique pharmaceutique comprimée selon l'une quelconque des revendications précédentes, comprenant le ou les excipients pharmaceutiquement acceptables comme désintégrant choisi dans le groupe comprenant l'hydroxypropylméthylcellulose, la carboxyméthylcellulose sodique réticulée, la croscarmellose, la crospovidone, l'hydroxypropylcellulose à faible substitution et le glycolate d'amidon sodique.

10. Forme galénique pharmaceutique comprimée selon l'une quelconque des revendications précédentes, dans laquelle un rapport de liant:désintégrant est compris entre 10:1 et 8:1.

11. Forme galénique pharmaceutique comprimée selon l'une quelconque des revendications précédentes, dans laquelle l'ingrédient pharmaceutique actif est le métoclopramide ou la mélatonine.

12. Forme galénique pharmaceutique comprimée selon l'une quelconque des revendications précédentes, dans laquelle l'ingrédient pharmaceutique actif est choisi dans le groupe comprenant la loratadine, le donépézil, la diphénhydramine, la pseudoéphédrine, le lansoprazole, la mirtazapine, le rizatriptan, l'ondansétron, le zolmitriptan, l'olanzapine, la morphine, l'érythromycine, le dompéridone, l'oméprazole, l'avanafil, le lodénafil, le mirodénafil, le sildénafil, le tadalafil, le vardénafil, l'udénafil, le zaprinast, le benzamidénafil, la nitroglycérine, l'œstrogène, la progestérone, l'adrénaline et le dasantafil.

13. Forme galénique pharmaceutique comprimée selon l'une quelconque des revendications précédentes, dans laquelle l'ingrédient pharmaceutique actif est le métoclopramide pour une utilisation dans la prévention, la prophylaxie ou le traitement d'une maladie, telle que la gastroparésie.

14. Forme galénique pharmaceutique comprimée selon l'une quelconque des revendications précédentes, dans laquelle l'ingrédient pharmaceutique actif est la mélatonine pour une utilisation dans la prévention, la prophylaxie ou le traitement d'une maladie, telle que les troubles de type insomnie.
